# EUROPÄISCHE PATENTANMELDUNG

(11) **EP 1 720 173 A1**
(43) Veröffentlichungstag der Anmeldung: **08.11.2006**
(21) Anmeldenummer: 05009871.4
(22) Anmeldetag: 06.05.2005
(51) Int. Cl.: G21K 1/04

(54) **Kollimator zum Begrenzen eines Bündels energiereicher Strahlen**

(71) Anmelder: Deutsches Krebsforschungszentrum Stiftung des öffentlichen Rechts, 69120 Heidelberg (DE)
(72) Erfinder: Echner, Gernot, 69257 Wiesenbach (DE)
(74) Vertreter: Weber, Walter

(57) **Zusammenfassung**

Die Erfindung betrifft einen Kollimator (1) zum Begrenzen eines Bündels energiereicher Strahlen (2), das von einer im wesentlichen punktförmigen Strahlungsquelle (3) ausgehend auf ein Behandlungsobjekt (4) gerichtet ist und insbesondere der stereotaktischen Konformationsbestrahlung von Tumoren dient, wobei der Kollimator (1) als strahlbegrenzendes Mittel eine Irisblende (5) aufweist.

Für einen solchen Kollimator (1) wird eine hohe Abschirmung bei geringer Bauhöhe und mit einer variablen Öffnungsgröße der Blendenöffnung (12) dadurch erzielt, daß die Irisblende (5) mindestens drei Blendenblätter (6, 6', 6" oder 7, 7', 7", 7"' oder 8, 8', 8", 8"', 8'''' oder 9, 9', 9", 9"', 9"", 9'"") aufweist, die gleiche Winkel (α) einschließende, aneinanderliegende Seitenflächen (10) aufweisen, wobei die Blendenblätter (6, 6', 6" oder 7, 7', 7'', 7''' oder 8, 8', 8", 8'", 8'''' oder 9, 9', 9", 9''', 9'''', 9''''') eine strahlbegrenzende Öffnung (12) dadurch freigeben, daß eine Schiebebewegung (13) entlang der Seitenflächen (10) durch eine höchstens um eines verminderte Anzahl von Blendenblättem (6, 6', 6" oder 7, 7', 7", 7"' oder 8, 8', 8", 8"', 8"" oder 9, 9', 9", 9"', 9'''', 9''''') stattfindet.

## Beschreibung

Die Erfindung betrifft einen Kollimator zum Begrenzen eines Bündels energiereicher Strahlen, das von einer im wesentlichen punktförmigen Strahlungsquelle ausgehend auf ein Behandlungsobjekt gerichtet ist und insbesondere der stereotaktischen Konformationsbestrahlung von Tumoren dient, wobei der Kollimator als strahlbegrenzendes Mittel eine Irisblende aufweist.

Kollimatoren zum Begrenzen eines Bündels energiereicher Strahlen werden zu Diagnosezwecken und zur Behandlung insbesondere von Tumoren eingesetzt. Dabei dienen die Kollimatoren zur Begrenzung der Strahlen, damit neben dem Diagnose- bzw. Behandlungsbereich liegendes gesundes Gewebe möglichst gut vor Strahlen geschützt wird, um Schäden zu vermeiden oder auf ein Minimum zu reduzieren. Diesem Zweck dienen wechselbare feste Kollimatoren oder Kollimatoren, deren Öffnungen einstellbar sind, wie dies beispielsweise bei Multileafkollimatoren der Fall ist.

Im RHHCT Journal, 17. Juni 2002 (http://www.rhhct.org.uk/news/rhhct17/17.html) wird unter der Überschrift "The Centenary of the founding of the British Electro Therapeutic Society 1902-2002" darüber berichtet, daß auf einem Meeting am 24.03.1905 der Vorschlag gemacht wurde, einen Kollimator der eingangs genannten Art, also mit einer Irisblende als strahlbegrenzendes Mittel, zu verwenden. Dabei sind auch die sehr dicken Blendenblätter wegen der notwendigen Abschirmeigenschaften erwähnt.

Da zu dieser Zeit nur Strahlungsquellen mit einer Leistung im Kilovoltbereich verwendet wurden, waren unter den erwähnten sehr dicken Blendenblättem solche zu verstehen, die in der Größenordnung einer Dicke von 1,5 bis 3 mm lagen. Bei dieser Dicke war der bekannte Aufbau von Irisblenden mit ringförmig angeordneten, sich in Teilbereichen überlappenden Blendenblättem noch möglich.

Heute werden insbesondere in der Strahlentherapie jedoch Strahlungsquellen eingesetzt, deren Leistung im Megavoltbereich liegt. Um derart energieintensive Strahlen in erforderlichem Maß abzuschirmen, sind jedoch Materialstärken von 6 bis 10 cm erforderlich. Wollte man eine Irisblende üblicher Bauart mit derart dicken, teilweise übereinanderliegenden Blendenblättem bauen, so würde eine Bauhöhe der Irisblende erreicht, die nicht mehr in vernünftiger Weise handhabbar ist.

Der Erfindung liegt daher die Aufgabe zugrunde, einen Kollimator der eingangs genannten Art derart auszubilden, daß bei hoher Abschirmung und geringer Bauhöhe, eine variable Öffnungsgröße der Blendenöffnung erzielbar ist.

Die Aufgabe wird erfindungsgemäß dadurch gelöst, daß die Irisblende mindestens drei Blendenblätter aufweist, die gleiche Winkel einschließende, aneinanderliegende Seitenflächen aufweisen, wobei die Blendenblätter eine strahlbegrenzende Öffnung dadurch freigeben, daß eine Schiebebewegung entlang der Seitenflächen durch eine höchstens um eines verminderte Anzahl von Blendenblättern stattfindet.

Der Grundgedanke der Erfindung besteht darin, eine Irisblende derart auszubilden, daß keinerlei Überlappung von Blendenblättem mehr erforderlich ist. Dies wird erreicht, indem alle Blendenblätter in einer Ebene liegen und deren Seitenflächen aneinanderliegen. Erst nach einer Schiebebewegung der Blendenblätter zur Herstellung der Blendenöffnung liegen kurze vordere Teilbereiche der Seitenflächen der Blendenblätter frei, um die auf diese Weise gebildete strahlbegrenzende Blendenöffnung zu begrenzen. Auf diese Weise wird es möglich, Irisblenden auch im Bereich sehr energiereicher Strahlen einzusetzen, wobei trotz der für die Strahlenabschirmung erforderlichen Dicke der Blendenblätter eine handhabbare Bauhöhe eingehalten werden kann. Da keine Überlappungsbereiche erforderlich sind, ist das Gewicht gegenüber einer üblichen Irisblendenkonstruktion geringer. Das Gewicht, welches im Wesentlichen durch das abschirmende Material bestimmt wird, entspricht ungefähr dem Gewicht von Festblenden. Der Aufbau ist relativ einfach und die für die Einstellung der Blendenöffnung erforderliche Stellbewegung läßt sich mit einfachen mechanischen oder elektronischen Mitteln erzielen. Die kompakte Bauweise erlaubt es auch, den mit der Irisblende ausgestatteten Kollimator durch entsprechende Vorrichtungen in die erforderlichen Positionen zu bringen. Dies ist insbesondere im Bereich der therapierenden Bestrahlung erforderlich für ein Abscannen einer Fläche oder für eine Bestrahlung, die aus unterschiedlichen Raumwinkeln auf das Behandlungsobjekt gerichtet ist. Dabei ist eine einstellbare Irisblende schneller auf die erforderliche Größe zu bringen, als dies durch den Wechsel von festen Blenden möglich ist. Gegenüber Blendenplatten mit mehreren in den Strahlengang bringbaren Öffnungen weist sie dagegen sowohl ein geringeres Gewicht, als auch eine höhere Flexibilität bezüglich der einstellbaren Blendenöffnungsweite auf.

Prinzipiell ist es möglich, daß ein Blendenblatt fest angeordnet ist und sich die anderen Blendenblätter zur Bildung der Öffnung um gleiche Teilstücke entlang der Kante eines angrenzenden Blendenblattes verschieben. Damit jedoch der Mittelpunkt der Blendenöffnung unabhängig von deren Größe immer an der gleichen Stelle ist, ist es zweckmäßig, wenn eine Schiebebewegung aller Blendenblätter um gleiche Stellwege erfolgt, so daß nach der Stellbewegung die Öffnung durch Teilbereiche der Seitenflächen gebildet wird, die gleiche Entfernungen vom Mittelpunkt aufweisen. Bei dieser Ausgestaltung bleibt die optische Achse unabhängig von der Öffnungsbewegung der Blendenblätter der Irisblende immer an derselben Stelle.

Was bezüglich der Lagerung der Blendenblätter erforderlich ist, hängt davon ab, ob alle Blendenblätter eine Schiebebewegung vollziehen und wie viele Blendenblätter vorhanden sind. Bei lediglich drei Blendenblättem, bei denen eines fest ist, reicht zur Führung der beiden verschiebbaren Blendenblätter eine sichere Anlage an den Seitenflächen des feststehenden Blendenblattes aus. Insbesondere wenn alle Blendenblätter verschiebbar sind, erfordert ein eindeutiger Bewegungsverlauf, daß die Blendenblätter mittels in Richtung der Schiebebewegung verlaufenden Linearführungen gelagert sind. Bezüglich des Verlaufs der Linearführungen muß der genaue Verlauf der Bewegung eines jeden Blendenblattes berücksichtigt werden. Dies ist aus der Figurenbeschreibung noch näher ersichtlich. Beispielsweise ergibt sich für eine vierblättrige Blende, daß die Linearführungen in einem 45°-Winkel zu den Seitenflächen verlaufen müssen, die an die anderen Blendenblätter anliegen.

Eine besonders zweckmäßige Ausgestaltung besteht darin, daß die Irisblende vier Blendenblätter aufweist. Dies ergibt eine quadratische Öffnung, welche in einer Scannbewegung derart über die Fläche eines Behandlungsobjekts geführt werden kann, daß am Ende des Scannvorgangs die Bestrahlungsdauer auf der gesamten bestrahlten Fläche exakt gleich ist.

Eine Irisblende mit vier Blendenblättem kann auch so ausgestaltet werden, daß die jeweilige, die Öffnung bildende Seitenfläche an ihrem inneren Ende in einen nasenartig auskragenden, einen Viertelkreis bildenden Kreisbogen übergeht, der dann den Abschluß dieser Seitenfläche bildet. Werden dann die vier Blendenblätter derart zusammengefügt, daß die Kreisbögen unmittelbar aneinanderliegen, so entsteht eine runde Öffnung. Eine solche ist insbesondere dann zweckmäßig, wenn für ein Scannen oder eine punktuelle Bestrahlung ein Einzelstrahl mit einem sehr kleinen Durchmesser benötigt wird. Werden derartige Blendenblätter weiter geöffnet, so entsteht eine quadratische Öffnung mit runden Ecken, wobei verschiedene Größen möglich sind. Diese kann dann in der oben genannten Art und Weise zum Scannen eingesetzt werden. Auf diese Weise läßt sich mit relativ großen quadratischen Öffnungen der Großteil einer Fläche abscannen und es ist möglich, mit der runden kleinen Öffnung einen sehr feinen Strahl zu erzielen, mit dem unregelmäßige Randbereiche, für die die quadratische Öffnung zu großflächig ist, noch abgescannt werden können.

Alternativ kann die Irisblende dafür eingerichtet sein, daß ein möglichst runder Strahl geformt wird. Dafür sind dann mindestens sechs Blendenblätter zweckmäßig, wobei sich mit der Anzahl natürlich die Annäherung an die Kreisform immer mehr verbessert. Dies ermöglicht es, eine große Öffnung zu bilden, wie sie beispielsweise für eine Röntgenbestrahlung zum Zweck der Diagnose erforderlich ist oder man kann mit energiereicheren Strahlen einen runden Tumor, wie beispielsweise einen Gehirntumor, bestrahlen. Andererseits kann man die Öffnung zuerst größer lassen, um eine Fläche abzuscannen und dann klein zu machen und mit einer Scannbewegung beliebige Formen, wie beispielsweise die unregelmäßigen Randbereiche der Fläche eines Behandlungsobjekts, abzuscannen.

Soll mittels des Kollimators ein Abscannen einer Fläche vorgenommen werden - dies ist die häufigste Anwendung -, so muß eine Scannvorrichtung vorgesehen sein, welche ein Behandlungsobjekt mittels der durch den Kollimator begrenzten Strahlen abscannt. Eine derartige Scannvorrichtung ist aus der deutschen Patentschrift DE 101 57 523 C1 bekannt. In diesem Fall tritt die Irisblende an die Stelle der dort offenbarten Kollimatoröffnungen mit festen Größen, die dort wahlweise in der erforderlichen Größe in den Strahlengang gebracht werden müssen. Alle übrigen in dieser Schrift offenbarten Merkmale lassen sich auf den Kollimator mit der Irisblende entsprechend übertragen, insbesondere die Mechanik um Strahlenquelle und Kollimator in der erforderlichen Scannbewegung zu führen. Deren Beschreibung wird daher durch diesen Verweis ersetzt. Eine andere Möglichkeit einer solchen Scannvorrichtung besteht darin, daß sich Strahlenquelle und Kollimator an einem Roboterarm befinden, der um das Behandlungsobjekt bewegbar ist. Derartige Roboterarme sind bekannt. Sie finden insbesondere in der automatisierten Fertigung bereits zahlreiche Verwendung, so daß auch auf deren nähere Beschreibung verzichtet werden kann.

Strahlenquelle und Kollimator können auch mittels einer Gantry in verschiedne Raumwinkelausrichtungen der durch den Kollimator begrenzten Strahlen zum Behandlungsobjekt bringbar sein. Selbstverständlich kann auch die oben genannte Scannvorrichtung in einer solchen Gantry aufgehängt werden, um ein zu bestrahlendes räumliches Gebilde von verschiedenen Seiten abzuscannen. Derartige Gantrys finden bereits in Bestrahlungsgeräten üblicherweise Verwendung, insbesondere im Zusammenhang mit Multileafkollimatoren, welche die zu bestrahlende Fläche mittels einer aufwendigen Mechanik nachbilden.

Bei dem erfindungsgemäßen Kollimator muß gewährleistet sein, daß die Seitenflächen der Blendenblätter der Irisblende exakt aufeinanderliegen. Sie müssen daher exakt plan sein und dürfen keine Oberflächenrauheit aufweisen. Dazu ist gegebenenfalls eine Feinstbearbeitung, wie zum Beispiel Schleifen oder Läppen erforderlich. Weiterhin ist notwendig, daß die Seitenflächen fest aneinanderliegen, wozu vorgeschlagen wird, daß Kraftbeaufschlagungen vorgesehen sind, die die Seitenflächen der Blendenblätter gegeneinander drücken. Beispielsweise kann vorgesehen sein, daß auf die Blendenblätter Federn wirken, die die Kraftbeaufschlagungen bewirken. Durch derartige Kraftbeaufschlagungen läßt sich eine noch bessere Flächenanlage als durch präzise Führungen erzielen, da Führungen immer ein kleines Spiel aufweisen müssen.

Eine weitere Möglichkeit für ein gutes Aneinanderliegen der Seitenflächen besteht darin, daß diese in ihren nicht zur Bildung der Blendenöffnung dienenden aneinanderliegenden Bereichen gemeinsame Führungen aufweisen, in denen die Seitenflächen der aneinandergrenzenden Blendenblätter gegenseitig verschiebbar sind. Auch eine derartige gemeinsame Führung zweier Seitenflächen aneinandergrenzender Blendenblätter kann eine Kraftbeaufschlagung durch Federn zum Zweck einer präzisen Flächenanlage der Seitenflächen beinhalten.

Die Schiebebewegung der Blendenblätter wird dadurch erreicht, daß mindestens ein Blendenblatt angetrieben wird. Dies reicht insbesondere bei der bereits erwähnten dreiblättrigen Irisblende aus. Sind mehr Blendenblätter vorhanden, so kann beispielsweise jedes zweite Blendenblatt angetrieben werden und die anderen Blendenblätter bewegen sich durch die dabei erzielte Kraftübertragung mit. Für einen möglichst reibungsfreien und exakten Bewegungsablauf der Öffnungsbewegung ist es jedoch zweckmäßig, wenn alle Blendenblätter simultan angetrieben werden.

Ein solcher simultaner Antrieb aller Blendenblätter kann auf unterschiedliche Weise erfolgen. Beispielsweise ist es möglich, daß für jedes Blendenblatt ein Antrieb vorgesehen ist, wobei durch eine elektronische Steuerung eine simultane Bewegung erfolgt. Diese muß allerdings sehr präzise sein, da ein ungleichmäßiger Antrieb dazu führen würde, daß sich die Blendenblätter gegenseitig verkeilen. Eine weitere Möglichkeit sieht vor, daß ein Antrieb über eine Mechanik alle Blendenblätter simultan antreibt. Derartige Mechaniken können auf unterschiedlichste Weise ausgebildet werden. So können beispielsweise Spindel- oder Schneckentriebe vorgesehen sein, die über ein Getriebe simultan bewegt werden. Eine weitere Möglichkeit besteht darin, daß die Mechanik eine um den Mittelpunkt der Blendenöffnung drehbare Kurvenscheibe aufweist, wobei natürlich eine Öffnung in der Mitte der Kurvenscheibe erforderlich ist, die einen Strahldurchtritt des größtmöglichen Strahls zuläßt. Auf dieser Kurvenscheibe sind dann spiralförmig Stellkurven angeordnet, welche die Blendenblätter betätigen. Bei den Stellkurven kann es sich um Nuten oder Erhöhungen handeln, welche die Blendenblätter durch Elemente verstellen, die an diesen angeordnet sind und auf den Stellkurven gleiten.

Eine weitere Möglichkeit des Aufbaus einer solchen Mechanik sieht vor, daß ein um den Mittelpunkte der Blendenöffnung drehbares Stellorgan vorgesehen ist, das auf jedes Blendenblatt mit einem Stellarm wirkt. Dann ist es natürlich zweckmäßig, wenn eine derartige Schiebebewegung mittels der Stellarme auch eine Rückstellung beinhaltet. Solche kann beispielsweise durch Rückstellfedern, die der Stellbewegung entgegenwirken, erfolgen.

Wie bereits erwähnt müssen die Seitenflächen der Blenden absolut planparallel aufeinanderliegen, da sonst ein Spalt entsteht, durch den Leckstrahlen hindurchtreten können, was vermieden werden muß, da derartige Leckstrahlen auf gesundes Gewebe treffen würden. Daß sich ein sehr geringer Spalt auch durch die präziseste Flächenbehandlung nicht völlig über den gesamten Flächenverlauf der aneinandergrenzenden Flächen vermeiden läßt, ist es zweckmäßig, wenn die aneinanderliegenden Seitenflächen derart verlaufen, daß der Spalt nicht parallel zum Strahlengang verläuft. Dazu kann vorgesehen sein, daß die Seitenflächen in Schieberichtung verlaufende komplementär ineinandergreifende Abweichungen bezüglich der Ebenheit der Seitenflächen aufweisen. Es ist bekannt, dafür Stufen oder ähnliches vorzusehen. Deshalb wird auf diese Ausgestaltungen nicht näher eingegangen. Eine gute Lösung zur Vermeidung der erwähnten Leckstrahlen besteht für die erfindungsgemäße Irisblende darin, daß diese zu einer rechtwinklig zur optischen Achse der Strahlen liegenden gedachten Kollimatorebene derart gekippt ist, daß ein Strahl nicht mehr durch einen möglichen Spalt fallen kann. Da ein solcher durch Toleranzen möglicher Spalt bei hoher Präzision der Flächen im Mikrometerbereich liegt, reicht eine entsprechend kleine Kippung um Bogensekunden aus, welche auf die Strahlenformung keine praktische Auswirkung hat.

Der erfindungsgemäße Kollimator kann natürlich außer im Bereich sehr energiereicher Strahlen auch für Röntgengeräte eingesetzt werden, da die Vorteile nicht überlappender Blendenblätter auch dort relevant sind, weil eine geringe Bauhöhe für jedes Gerät von Vorteil ist. Der Zweck der Erfindung besteht natürlich insbesondere darin, eine Abschirmungsfähigkeit für besonders energiereiche Strahlen bei geringer Bauhöhe einer Irisblende verfügbar zu machen. Für solche Anwendungsbereiche sollte die Abschirmungsfähigkeit für energiereiche Strahlen bezüglich einer Strahlungsquelle im Megavoltbereich ausgelegt sein. Dies betrifft dann hauptsächlich den Einsatzbereich der Strahlentherapie, da beispielsweise für die Zerstörung von Tumorgeweben solche energiereichen Strahlen erforderlich sind. Für diesen Zweck sollte die Dicke der Blendenblätter zwischen 6 und 10 cm liegen, wobei übliches Blendenblattmaterial vorausgesetzt wird, das beispielsweise eine Wolframlegierung sein kann.

Um eine zusätzliche Abschirmung zu erzielen, kann vorgesehen sein, daß der erfindungsgemäße Kollimator außer der Irisblende eine Festblende aufweist, die sich im Strahlengang befindet und deren Öffnung auf die größtmögliche Öffnung der Irisblende abgestimmt ist.

Die Erfindung wird nachfolgend anhand von in der Zeichnung dargestellten Ausführungsbeispielen erläutert. Es zeigen
- **Fig. 1**: ein einfaches Ausführungsbeispiel einer dreiblättrigen Irisblende zur Erläuterung des Prinzips,
- **Fig. 2**: eine Prinzipskizze des Kollimators,
- **Fig. 3**: eine Führung an den Seitenflächen von Blendenblättern,
- **Fig. 4a, 4b und 4c**: eine Prinzipskizze einer vierblättrigen Irisblende,
- **Fig. 5a, 5b und 5c**: ein Ausführungsbeispiel einer Mechanik zur simultanen Verstellung von Blendenblätter,
- **Fig. 6a und 6b**: eine weitere Ausgestaltungsmöglichkeit einer vierblättrigen Irisblende,
- **Fig. 7a, 7b und 7c**: eine Prinzipskizze einer fünfblättrigen Irisblende,
- **Fig. 8a, 8b und 8c**: eine Prinzipskizze einer sechsblättrigen Irisblende,
- **Fig. 9**: ein weiteres Ausführungsbeispiel einer Mechanik zur simultanen Verstellung von Blendenblättern und
- **Fig. 9a**: eine Einzelheit dieser Mechanik.

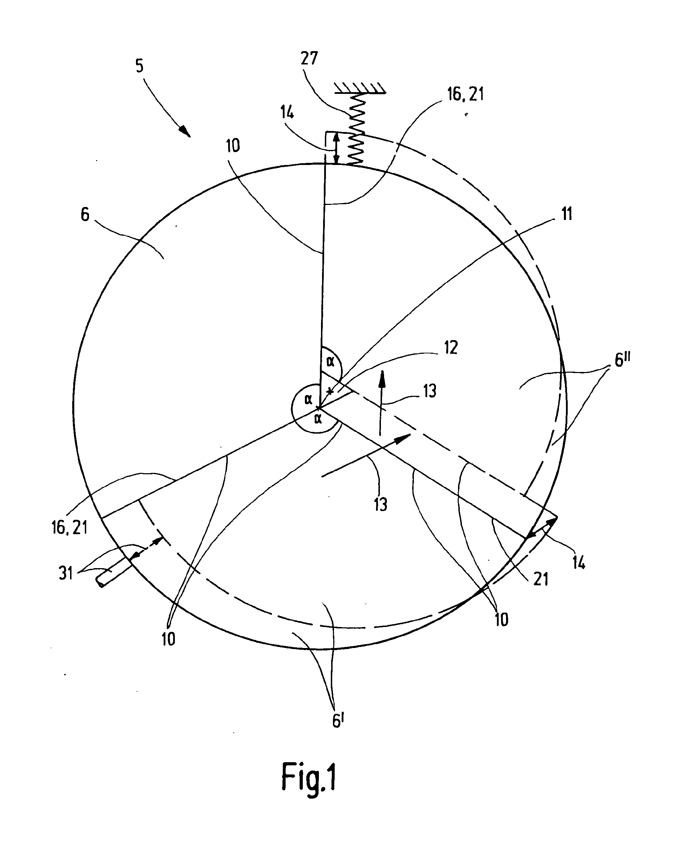

**Fig. 1** zeigt ein einfaches Ausführungsbeispiel einer dreiblättrigen Irisblende 5 zur Erläuterung des Prinzips. Für diese Erläuterung wurde eine dreiblättrige Irisblende 5 deshalb gewählt, da sie aufgrund der wenigen Teile am übersichtlichsten darstellbar ist. Diese Irisblende 5 verfügt über drei Blendenblätter 6, 6' und 6". Bei diesem Ausführungsbeispiel ist das Blendenblatt 6 fest und die Blendenblätter 6' und 6" in Richtung der Pfeile 13 verschiebbar. Im geschlossenen Zustand der Irisblende 5 befmden sich die Winkel α am Mittelpunkt 11, wobei jeder Winkel α durch zwei Seitenflächen 10 der Blendenblätter 6, 6', 6" gebildet ist. Diese Winkel α fallen natürlich bei Irisblenden 5 mit mehr Blendenblättem in entsprechender Weise kleiner aus.

Bei dieser Ausführungsform sind die Blendenblätter 6' und 6" mittels Führungen 21 an den Seitenflächen 10 derart geführt, daß die Seitenflächen 10 fest aufeinanderliegen. Dabei bilden aufgrund der festen Anordnung des Blendenblattes 6 dessen Seitenkanten 10 Linearführungen 16 für die beiden anderen Blendenblätter 6' und 6" und die Führung 21 zwischen den beiden letztgenannten verschiebt sich mit diesen Blendenblättem 6' und 6", wobei diese die Stellwege 14 vollziehen. Dieser Verschiebung dient ein symbolisch dargestellter Antrieb 31 am Blendenblatt 6' und der Rückstellung dient eine Rückstellfeder 27 am Blendenblatt 6". Durch die Schiebebewegung 13 wird eine Blendenöffnung 12 geöffnet, wobei sich die Größe der Blendenöffnung 12 nach dem Maß der zurückgelegten Stellwege 14 richtet.

Günstiger als eine dreieckige Blendenöffnung 12 ist eine viereckige oder eine nahezu runde Form. Dies zeigen später dargestellte und beschriebene Ausführungsbeispiele. Weiterhin ist eine Ausgestaltung zweckmäßiger, bei der sich der Mittelpunkt 11 mit der Öffnung der Irisblende 5 nicht verschiebt, wie dies hier mit den beiden kleinen Kreuzen eingezeichnet ist, sondem bei denen dieser Mittelpunkt 11 erhalten bleibt, wenn sich die Irisblende 5 öffnet. Zu diesem Zweck müssen dann jedoch alle Blendenblätter eine Schiebebewegung 13 vollziehen und es ist daher erforderlich, daß diese Blendenblätter mittels entsprechender Linearführungen gelagert sind. Auch dies wird an den noch folgenden Ausführungsbeispielen erläutert.

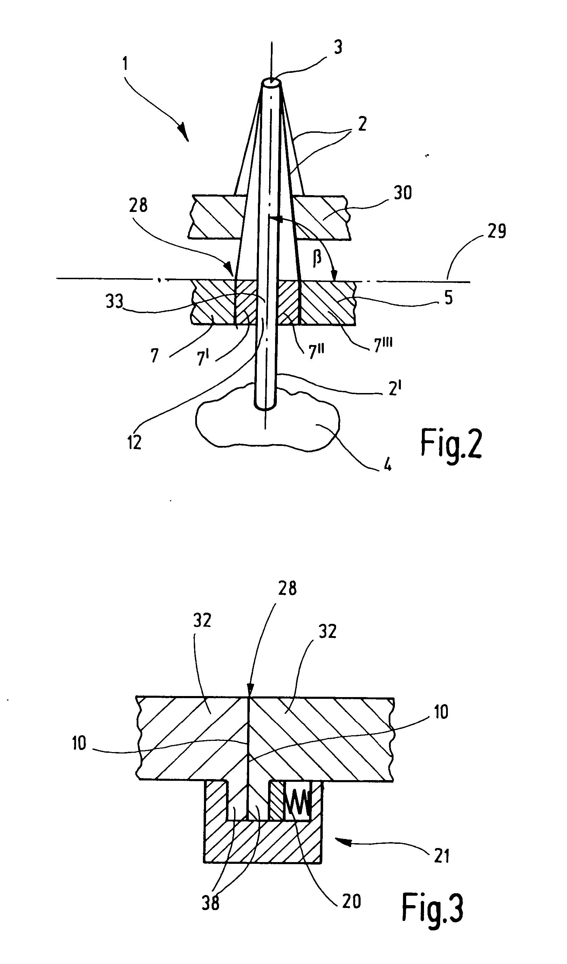

**Fig. 2** zeigt eine Prinzipskizze des Kollimators 1 mit einer Irisblende 5. Der Kollimator 1 ist einer Strahlungsquelle 3 zugeordnet, von der Strahlen 2 ausgehen. Der Irisblende 5 vorgeordnet ist eine Festblende 30, die eine Öffnung aufweist, welche der größtmöglichen Öffnung der Irisblende 5 entspricht. Diese Festblende 30 dient dazu, die Strahlen 2 der Strahlungsquelle 3 einzugrenzen und das Auftreten von Streustrahlen so weit wie möglich zu verhindern. Die Irisblende 5 ist hier in einer Schnittdarstellung gezeigt, wobei es sich um eine vierblättrige Irisblende 5 mit Blendenblättem 7, 7', 7", 7"' handelt. Diese wird später noch näher dargestellt. Durch die Öffnung 12 der Irisblende 5 werden die Strahlen 2 auf die Strahlen 2' weiter eingeengt, und zwar derart, daß mit diesen Strahlen 2' beispielsweise die Fläche eines Behandlungsobjekts 4 abgescannt werden kann, wobei bezüglich einer solchen Scannvorrichtung auf die DE 101 57 523 Cl verwiesen wird. Eine solche Scannvorrichtung kann wiederum auf einer Gantry angeordnet sein, so daß es möglich ist, das Behandlungsobjekt 4 von unterschiedlichen Seiten aus zu bestrahlen und so eine maximale Bestrahlung, beispielsweise eines Tumors zu erzielen, bei der gleichzeitig das umliegende Gewebe wesentlich weniger Strahlung erhält.

**Fig. 3** zeigt eine Einzelheit einer Führung 21 zwischen zwei Blendenblättem 32. Die Blendenblätter 32 können beliebige Blendenblätter sein, dergestalt wie sie in dieser Beschreibung vorhanden sind oder natürlich auch einer Irisblende 5, die noch mehr Blendenblätter aufweist. Die hier dargestellte Führung 21 dient dazu, zwei Blendenblätter 32 mit ihren Seitenflächen 10 derart fest zusammenzuhalten, daß möglichst kein Spalt 28 entsteht. Dazu dient auch die in der Führung 21 angeordnete Feder 20, welche an die Blendenblätter 32 angefügte Absätze 38 zusammenpreßt. Solche Führungen 21 können natürlich nur in den Bereichen der Seitenflächen 10 angeordnet sein, die nicht als Teilbereiche 15 für die Bildung einer Blendenöffnung 12 herangezogen werden.

Da sich ein Spalt 28 nie hundertprozentig vermeiden läßt, wird vorgeschlagen, daß die in **Fig. 2** eingezeichnete Blendenebene 29 zu der optischen Achse 33 leicht gekippt wird, und zwar derart, daß keine Strahlen 2 durch einen Spalt 28 zwischen Blendenblättern 32 fallen können. Dies heißt, daß der Winkel β eine leichte Abweichung von 90° hat, wobei in der Regel wenige Bogensekunden ausreichend sind. Ergänzend ist hinzuzufügen, daß der Strahlengang in Fig. 2 wesentlich verkürzt gezeichnet ist. Tatsächlich ist im Verhältnis zur Blendenöffnung 12 der Abstand zur Strahlungsquelle 3 wesentlich größer, so daß die Strahlen 2 im Bereich der Irisblende 5 nur noch eine minimale Abweichung von einem parallelen Verlauf haben, so daß im Gegensatz zur Darstellung der Fig. 2 ein Hindurchtreten von Strahlen 2 durch einen Spalt 28 möglich ist und daher durch die erwähnte Verkippung oder eine andere Maßnahme unterbunden werden sollte. Die Verkippung kann jedoch sehr gering ausfallen, weil aufgrund der hohen Oberflächenqualität und Planheit der Seitenflächen 10 ein Spalt 28 ohnehin nur im Mikrometerbereich auftreten kann.

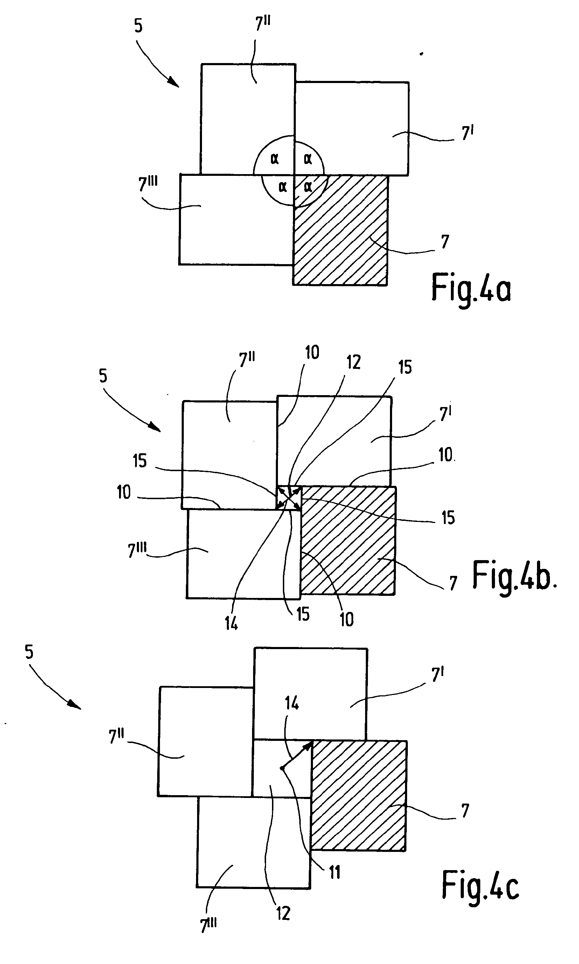

Die **Fig. 4a, 4b und 4c** zeigen eine Prinzipskizze einer vierblättrigen Irisblende 5. Diese verfügt über die Blendenblätter 7, 7', 7"und 7"'. In **Fig. 4a ist** die Irisblende 5 geschlossen, wobei die Winkel α, die rechtwinklig sind, aneinanderliegen. In **Fig. 4b** haben sich alle Blendenblätter um denselben Stellweg 14 bewegt, so daß eine Öffnung 12 entstanden ist, welche von Teilbereichen 15 der Seitenflächen 10 der Blendenblätter 7, 7', 7", 7"' gebildet wird. Die Stellwege 14 entsprechen jeweils der Hälfte der beiden Diagonalen der quadratischen Öffnung 12.

**Fig. 4c** zeigt eine weitere Öffnung der Irisblende 5, wobei der Mittelpunkt 11, der in der optischen Achse 33 (siehe Fig. 2) liegt, eingezeichnet ist, und von diesem ausgehend der Stellweg 14, den das linke obere Eck des Blendenblatts 7 vollzogen hat. In entsprechender Weise haben auch die anderen Blendenblätter 7', 7" und 7'" Stellwege 14 vollzogen.

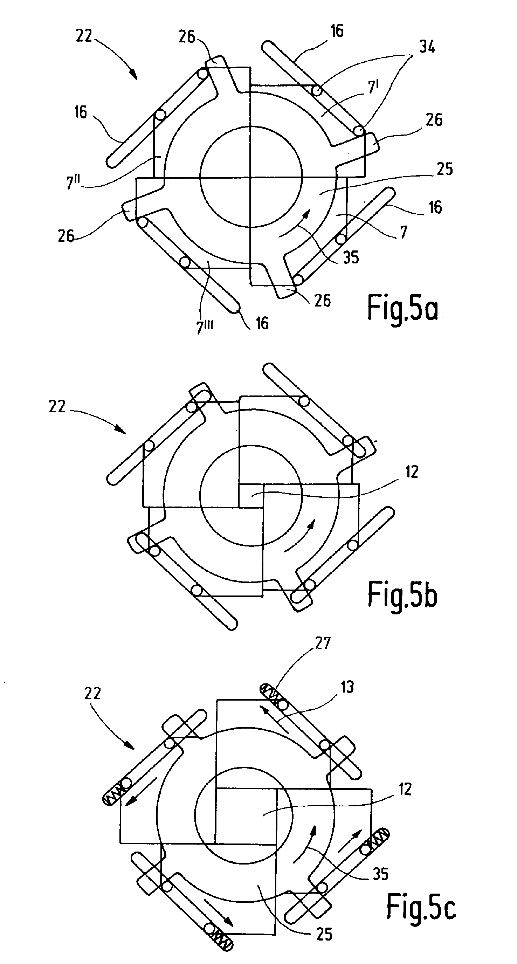

Die **Fig. 5a, 5b und 5c** zeigen ein Ausführungsbeispiel einer Mechanik 22 zur simultanen Verstellung von Blendenblättem. Dargestellt ist dies anhand von vier Blendenblättern 7, 7', 7" und 7"'. Die Mechanik 22 weist ein Stellorgan 25 auf, das über Stellarme 26 verfügt, welche jeweils einem der Blendenblätter 7, 7', 7" und 7'" zugeordnet sind. Die Blendenblätter 7, 7', 7" und 7'" besitzen Führungsbolzen 34 und zwar jeweils zwei, welche in Linearführungen 16 gelagert sind. Wenn sich das Stellorgan 25 in Richtung des Pfeils 35 dreht, schieben die Stellarme 26 die Bolzen 34 entlang der Linearführungen 16 und bewirken somit die oben beschriebenen Stellwege 14 der Blendenblätter 7, 7', 7", 7"'.

**Fig. 5b** zeigt bereits eine Öffnung 12, die in **Fig. 5c** noch weiter geöffnet ist. In **Fig. 5c** sind auch die Schiebebewegungen 13 eingezeichnet, sowie die mögliche Anordnung von Rückstellfedern 27, welche die Irisblende 5 wieder schließen, wenn das Stellorgan 25 entgegen der Richtung des Pfeils 35 zurückbewegt wird.

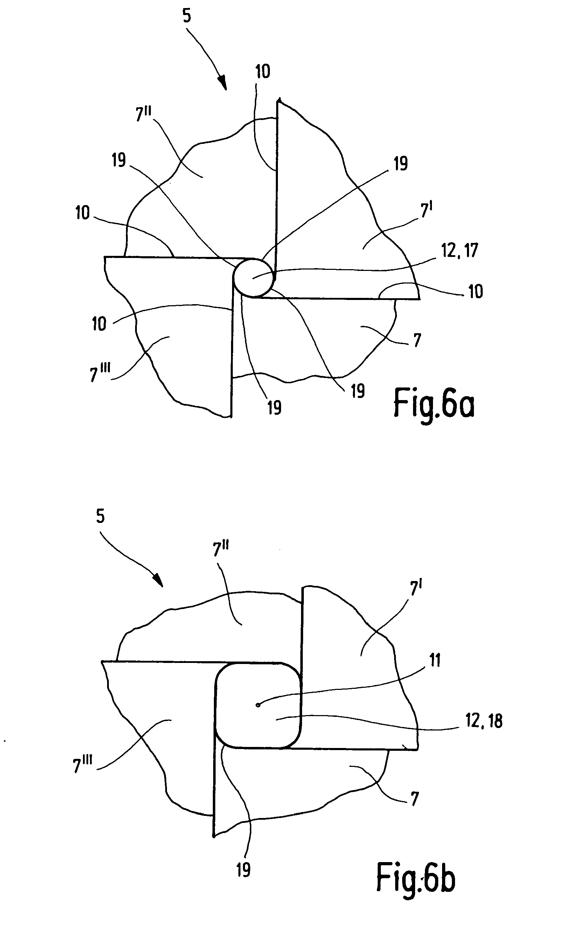

Die **Fig. 6a und 6b** zeigen eine weitere Ausgestaltungsmöglichkeit einer vierblättrigen Irisblende 5. Dabei weisen die Blendenblätter 7, 7', 7" und 7"' an den vorderen Enden ihrer Seitenflächen 10 nasenartig auskragende Kreisbögen 19 auf. Bei dieser Ausgestaltungsmöglichkeit kann die Irisblende 5 nicht völlig geschlossen werden, da eine Stellbewegung 13 hier nur soweit möglich ist, bis die Kreisbögen 19, die jeweils ein Vierteilkreis sind, sich zu einer runden Öffnung 17 zusammenfügen. Dies ist dann die kleinstmögliche Öffnung 12. Wird diese Irisblende 5 in entsprechender Weise wie oben beschrieben geöffnet, so entsteht eine quadratische Öffnung 18, bei der die Kreisbögen 19 gerundete Ecken bilden. Dies ist in **Fig. 6b** gezeigt. Auf die Vorteile einer derartigen Ausgestaltung wurde oben bereits verwiesen.

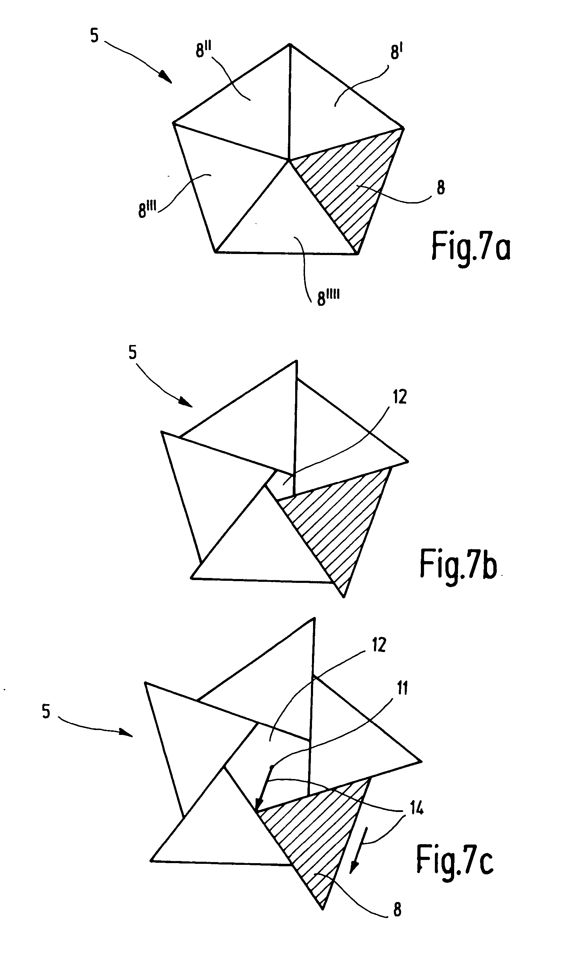

Die **Fig. 7a, 7b und 7c** zeigen eine Prinzipskizze einer fünfblättrigen Irisblende 5. Auch hier vollziehen alle Blendenblätter 8, 8', 8", 8'" und 8"" simultane Stellbewegungen, um eine Öffnung 12 zu bilden, wie sie in den **Fig. 7b und 7c** dargestellt ist. In der **Fig. 7c** ist noch verdeutlicht, welchen Stellweg 14 beispielsweise das schraffiert hervorgehobene Blendenblatt 8 vollzieht, wobei der vom Mittelpunkt 11 ausgehende Stellweg 14 den Weg beschreibt, den die Ecke des Blendenblatts 8 vollzogen hat, die jetzt an der Pfeilspitze liegt.

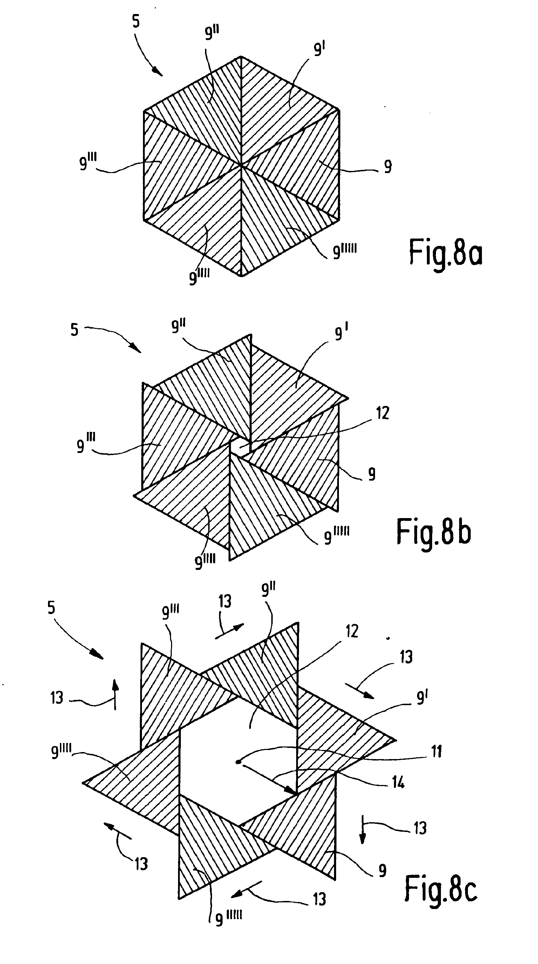

**Fig. 8a, 8b und 8c** zeigen eine Prinzipskizze einer sechsblättrigen Irisblende 5. Die Darstellungen entsprechen den zuvor erläuterten Darstellungen, wobei die Blendenblätter 9, 9', 9", 9"', 9"" und 9""' in verschiedenen Schraffuren gezeichnet sind, damit die Positionen dieser Blendenblätter bei den mit den **Fig. 8b und 8c** gezeigten Öffnungsbewegungen besser erkennbar sind. In **Fig. 8c** ist noch mit den Pfeilen 13 die Schiebebewegung eines jeden der Blendenblätter 9, 9', 9", 9"', 9'"' und 9""' eingezeichnet. Die Stellbewegung 14 ist mit einem vom Mittelpunkt 11 ausgehenden Pfeil für das Eck an der Pfeilspitze dargestellt, das zum Blendenblatt 9' gehört.

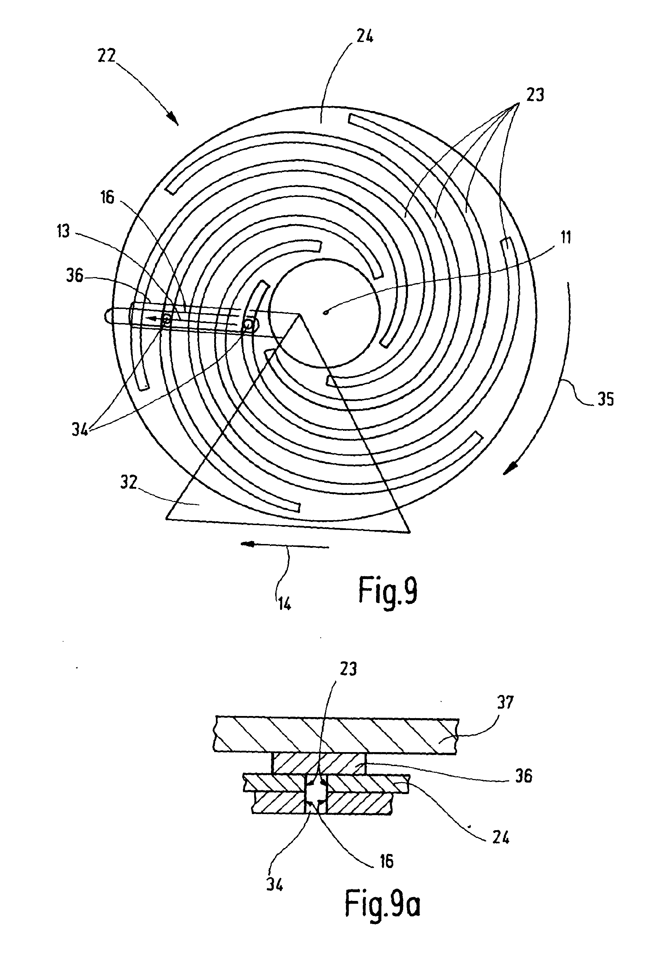

Die **Fig. 9** zeigt ein weiteres Ausführungsbeispiel einer Mechanik 22 zur simultanen Verstellung von Blendenblättem, wobei die **Fig. 9a** eine Einzelheit dieser Mechanik 22 als Schnitt quer zu einem Haltearm 36 zeigt. Es handelt sich bei dem Ausführungsbeispiel um eine Kurvenscheibe 24, welche Stellkurven 23 aufweist. Die Blendenblätter 32 - es kann sich um beliebig ausgestaltete handeln - sind hier jeweils mit einem Haltearm 36 ausgestattet, welcher jeweils zwei Führungsbolzen 34 trägt. Dabei wurde die Darstellung auf ein Blendenblatt 32 beschränkt. Die Führungsbolzen 34 erstrecken sich durch die als Nuten ausgebildeten Stellkurven 23 der Kurvenscheibe 24 hindurch und laufen zusätzlich in Linearführungen 16. Auf diese Weise wird mittels einer Drehung in Richtung des Pfeils 35 der Kurvenscheibe 24 erreicht, daß das Blendenblatt 32 eine Schiebebewegung in Richtung des Pfeils 13 vollzieht. Bewegt sich dagegen die Kurvenscheibe 24 entgegen des Pfeils 35, so wird das Blendenblatt 32 wieder entgegen dem Stellweg 14 zurückgestellt. Diese Stellwege 14 vollziehen dann alle angeordneten Blendenblätter 32 immer in simultaner Weise.

Es wurde hier nicht festgelegt, wie viele Blendenblätter vorhanden sind, da derartige Kurvenscheiben 24 für eine nahezu beliebige Anzahl von Blendenblättern 32 eingesetzt werden können. Entsprechend der Anzahl der Blendenblätter 32 und entsprechend der gewünschten Öffnung 12 und damit der gewünschten Stellwege 14 richtet sich die Anzahl und der Verlauf der Stellkurven 23. Dabei zeigt die **Fig. 9a** noch eine Deckplatte 37, die die Mechanik 22 zweckmäßigerweise abdeckt. Statt der Haltearme 36 kann natürlich auch eine entsprechende Anordnung der Führungsbolzen 34 direkt am jeweiligen Blendenblatt 32 vorgenommen werden.

Die dargestellten Ausführungsbeispiele geben lediglich einen kleinen Ausschnitt von Möglichkeiten wieder. Insbesondere Lagerung und Stellmechanik kann auch auf andere Weise ausgebildet sein, wie dies eingangs bereits erwähnt wurde. Insbesondere ist es auch möglich, die Anzahl der Blendenblätter noch zu vergrößern, um eine möglichst runde Ausgestaltung der Blendenöffnung 12 zu erzielen. Auch könnten z. B. anstatt der Führungen 21 an den Seitenflächen 10 Schwalbenschwanzführungen vorgesehen sein, wenn diese nur in den Bereichen angeordnet sind, die nicht zur Bildung der Öffnung 12 dienen. Auch die Federn 20 könnten an den Außenseiten der Blendenblätter 6, 6', 6" oder 7, 7', 7", 7"' oder 8, 8', 8", 8"', 8"" oder 9, 9', 9", 9"', 9"", 9'"'' angeordnet sein, um sie jeweils gegen die Seitenflächen 10 der beiden angrenzenden Blendenblätter zu drücken. Eine weitere Möglichkeit, einen guten gegenseitigen Halt der Seitenflächen 10 zu erzielen, wäre eine elastische Umfassung aller Blendenblätter einer Irisblende 5. Zahlreiche weitere Ausgestaltungsmöglichkeiten sind denkbar.

### Bezugszeichenliste

- 1: Kollimator
- 2: Strahlen
- 2': Strahlen durch den Kollimator begrenzt
- 3: Strahlungsquelle
- 4: Behandlungsobjekt (Diagnose- oder Strahlentherapie)
- 5: Irisblende
- 6, 6', 6": Blendenblätter bei einer dreiblättrigen Irisblende
- 7, 7', 7", 7'": Blendenblätter bei einer vierblättrigen Irisblende
- 8,8',8", 8"', 8"": Blendenblätter bei einer fünfblättrigen Irisblende
- 9, 9', 9", 9'", 9"": Blendenblätter bei einer sechsblättrigen Irisblende
- 10: Seitenflächen
- 11: Mittelpunkt
- 12: Öffnung / Blendenöffnung
- 13: Pfeile: Schiebebewegung
- 14: Stellwege
- 15: Teilbereiche der Seitenflächen, die die Öffnung bilden
- 16: Linearführungen
- 17: runde Öffnung
- 18: quadratische Öffnung mit gerundeten Ecken
- 19: Kreisbögen (nasenartig auskragend)
- 20: Federn
- 21: Führungen an den Seitenflächen
- 22: Mechanik
- 23: Stellkurven
- 24: Kurvenscheibe
- 25: Stellorgan
- 26: Stellarme
- 27: Rückstellfedern
- 28: Spalt (durch Toleranzen bedingt)
- 29: Blendenebene
- 30: Festblende
- 31: Antrieb (symbolisch)
- 32: beliebige Blendenblätter
- 33: optische Achse
- 34: Führungsbolzen an den Blendenblättem
- 35: Pfeil: Drehrichtung Kurvenscheibe oder Stellorgan
- 36: Haltearm eines Blendenblattes
- 37: Deckplatte
- 38: Absätze

- α: Winkel zwischen den Seitenflächen der Blendenblätter
- β: Winkel zwischen optischer Achse und Blendenebene

## Patentansprüche

1. Kollimator (1) zum Begrenzen eines Bündels energiereicher Strahlen (2), das von einer im wesentlichen punktförmigen Strahlungsquelle (3) ausgehend auf ein Behandlungsobjekt (4) gerichtet ist und insbesondere der stereotaktischen Konformationsbestrahlung von Tumoren dient, wobei der Kollimator (1) als strahlbegrenzendes Mittel eine Irisblende (5) aufweist,
**dadurch gekennzeichnet,**
**daß** die Irisblende (5) mindestens drei Blendenblätter (6, 6', 6" oder 7, 7', 7", 7'" oder 8, 8', 8", 8"', 8"" oder 9, 9', 9", 9"', 9"", 9""') aufweist, die gleiche Winkel (α) einschließende, aneinanderliegende Seitenflächen (10) aufweisen, wobei die Blendenblätter (6, 6', 6" oder 7, 7', 7", 7"' oder 8, 8', 8", 8"', 8'''' oder 9,9',9",9"',9"",9""') eine strahlbegrenzende Öffnung (12) dadurch freigeben, daß eine Schiebebewegung (13) entlang der Seitenflächen (10) durch eine höchstens um eines verminderte Anzahl von Blendenblättern (6, 6', 6" oder 7, 7', 7", 7'" oder 8, 8', 8", 8'", 8"" oder 9, 9', 9", 9'", 9"", 9'"") stattfindet.

2. Kollimator nach Anspruch 1,
**dadurch gekennzeichnet,**
**daß** eine Schiebebewegung (13) aller Blendenblätter (6, 6', 6" oder 7, 7', 7", 7'" oder 8, 8', 8", 8"', 8"" oder 9, 9', 9", 9"', 9"", 9""') um gleiche Stellwege (14) erfolgt, so daß nach der Stellbewegung die Öffnung (12) durch Teilbereiche (15) der Seitenflächen (10) gebildet wird, die gleiche Entfernungen vom Mittelpunkt (11) aufweisen.

3. Kollimator nach Anspruch 1 oder 2,
**dadurch gekennzeichnet,**
**daß** die Blendenblätter (6, 6', 6" oder 7, 7', 7", 7"' oder 8, 8', 8", 8"', 8"" oder 9, 9', 9", 9"', 9"", 9""') mittels in Richtung der Schiebebewegung (13) verlaufender Linearführungen (16) gelagert sind.

4. Kollimator nach Anspruch 1, 2 oder 3,
**dadurch gekennzeichnet,**
**daß** die Irisblende (5) vier Blendenblätter (7, 7', 7", 7"') aufweist.

5. Kollimator nach Anspruch 4,
**dadurch gekennzeichnet,**
**daß** die jeweilige, die Öffnung (12) bildende Seitenfläche (10) an ihrem inneren Ende in einen nasenartig auskragenden einen Viertelkreis bildenden Kreisbogen (19) übergeht, so daß die vier Blendenblätter (7, 7', 7", 7"') wahlweise eine runde Öffnung (17) oder quadratische Öffnungen mit gerundeten Ecken (18) verschiedenen Größen bilden können.

6. Kollimator nach Anspruch 1, 2 oder 3,
**dadurch gekennzeichnet,**
**daß** die Irisblende (5) mindestens sechs Blendenblätter (9, 9', 9", 9''', 9"", 9""') aufweist.

7. Kollimator nach einem der Ansprüche 1 bis 6,
**dadurch gekennzeichnet,**
**daß** eine Scannvorrichtung vorgesehen ist, welche ein Behandlungsobjekt (4) mittels der durch den Kollimator (1) begrenzten Strahlen (2') abscannt.

8. Kollimator nach einem der Ansprüche 1 bis 7,
**dadurch gekennzeichnet,**
**daß** sich Strahlenquelle (3) und Kollimator (1) an einem Robotterarm befinden, der um das Behandlungsobjekt (4) bewegbar ist.

9. Kollimator nach einem der Ansprüche 1 bis 7,
**dadurch gekennzeichnet,**
**daß** Strahlenquelle (3) und Kollimator (1) mittels einer Gantry in verschiedene Raumwinkelausrichtungen der durch den Kollimator (1) begrenzten Strahlen (2') zum Behandlungsobjekt (4) bringbar ist.

10. Kollimator nach einem der Ansprüche 1 bis 9,
**dadurch gekennzeichnet,**
**daß** Kraftbeaufschlagungen vorgesehen sind, die die Seitenflächen (10) der Blendenblätter (6, 6', 6" oder 7, 7', 7", 7'" oder 8, 8', 8", 8"', 8"" oder 9, 9', 9", 9"', 9"", 9""') gegeneinanderdrücken.

11. Kollimator nach Anspruch 10,
**dadurch gekennzeichnet,**
**daß** auf die Blendenblätter (6, 6', 6" oder 7, 7', 7", 7'" oder 8, 8', 8", 8"', 8"" oder 9, 9', 9", 9"', 9"", 9""') Federn (20) wirken, die die Kraftbeaufschlagungen bewirken.

12. Kollimator nach Anspruch 10 oder 11,
**dadurch gekennzeichnet,**
**daß** die Seitenflächen (10) in ihren nicht zur Bildung einer Öffnung (12) dienenden aneinanderliegenden Bereichen gemeinsame Führungen (21) aufweisen, in denen die Seitenflächen (10) der aneinandergrenzenden Blendenblätter (6, 6', 6" oder 7, 7', 7", 7'" oder 8, 8', 8", 8"', 8"" oder 9, 9', 9", 9"', 9"", 9""') gegenseitig verschiebbar sind.

13. Kollimator nach einem der Ansprüche 1 bis 12,
**dadurch gekennzeichnet,**
**daß** die Schiebebewegung (13) der Blendenblätter (6, 6', 6" oder 7, 7', 7", 7''' oder 8, 8', 8", 8"', 8"" oder 9, 9', 9", 9"', 9"", 9""') dadurch erfolgt, daß mindestens ein Blendenblatt (6, 6' oder 6"; 7, 7', 7" oder 7"'; 8, 8', 8", 8'" oder 8""; 9, 9', 9", 9"', 9"" oder 9""') angetrieben wird.

14. Kollimator nach Anspruch 13,
**dadurch gekennzeichnet,**
**daß** alle Blendenblätter (6, 6', 6" oder 7, 7', 7", 7"' oder 8, 8', 8", 8"', 8"" oder 9, 9', 9", 9"', 9"", 9""') simultan angetrieben werden.

15. Kollimator nach Anspruch 14,
**dadurch gekennzeichnet,**
**daß** für jedes Blendenblatt (6, 6', 6" oder 7, 7', 7", 7"' oder 8, 8', 8", 8"', 8"" oder 9, 9', 9", 9"', 9"", 9""') ein Antrieb vorgesehen ist, wobei durch eine elektronische Steuerung eine simultane Bewegung erfolgt.

16. Kollimator nach Anspruch 14,
**dadurch gekennzeichnet,**
**daß** ein Antrieb über eine Mechanik (22) alle Blendenblätter (6, 6', 6" oder 7, 7', 7", 7"' oder 8, 8', 8", 8"', 8"" oder 9, 9', 9", 9"', 9"", 9""') simultan antreibt.

17. Kollimator nach Anspruch 16,
**dadurch gekennzeichnet,**
**daß** die Mechanik (22) die Blendenblätter (6, 6', 6" oder 7, 7', 7", 7"' oder 8, 8', 8", 8"', 8"" oder 9, 9', 9", 9"', 9"", 9""') über, auf einer um den Mittelpunkt (11) drehbaren Kurvenscheibe (24) spiralförmig angeordnete Stellkurven (23) antreibt.

18. Kollimator nach Anspruch 16,
**dadurch gekennzeichnet,**
**daß** die Mechanik (22) ein um den Mittelpunkt (11) drehbares Stellorgan (25) aufweist, das auf jedes Blendenblatt (6, 6', 6" oder 7, 7', 7", 7'" oder 8, 8', 8", 8"', 8"" oder 9, 9', 9", 9"', 9"", 9""') mit einem Stellarm (26) wirkt.

19. Kollimator nach Anspruch 18,
**dadurch gekennzeichnet,**
**daß** der Schiebebewegung (13) mittels der Stellarme (26) Rückstellfedern (27) entgegenwirken.

20. Kollimator nach einem der Ansprüche 1 bis 19,
**dadurch gekennzeichnet,**
**daß** die aneinanderliegenden Seitenflächen (10) der Blenden (6, 6', 6" oder 7, 7', 7", 7"' oder 8, 8', 8", 8"', 8"" oder 9, 9', 9", 9"', 9"", 9""') derart verlaufen, daß ein durch Toleranzen bedingter Spalt (28) nicht parallel zum Strahlengang verläuft.

21. Kollimator nach Anspruch 20,
**dadurch gekennzeichnet,**
**daß** die Seitenflächen (10) in Schieberichtung verlaufende komplementär ineinandergreifende Abweichungen bezüglich der Ebenheit der Seitenflächen (10) aufweisen.

22. Kollimator nach Anspruch 20,
**dadurch gekennzeichnet,**
**daß** die Irisblende (5) zu einer rechtwinklig zur optischen Achse liegenden gedachten Blendenebene (29) derart gekippt ist, daß ein Strahl (2) nicht mehr durch einen Spalt (28) fallen kann.

23. Kollimator nach einem der Ansprüche 1 bis 22,
**dadurch gekennzeichnet,**
**daß** seine Abschirmungsfähigkeit für energiereiche Strahlen einer Strahlungsquelle (3) im Megavoltbereich ausgelegt ist.

24. Kollimator nach Anspruch 23,
**dadurch gekennzeichnet,**
**daß** die Dicke der Blendenblätter (6, 6', 6" oder 7, 7', 7", 7'" oder 8, 8', 8", 8'" 8"" oder 9, 9', 9", 9'", 9"", 9"'") zwischen 6 und 10 cm liegt .

25. Kollimator nach einem der Ansprüche 1 bis 24,
**dadurch gekennzeichnet,**
**daß** sich außer der Irisblende (5) eine Festblende (30) für eine zusätzliche Abschirmung im Strahlengang befindet, deren Öffnung auf die größtmögliche Öffnung (12) der Irisblende (5) abgestimmt ist.

## Geänderte Patentansprüche

### Geänderte Patentansprüche gemäss Regel 86(2) EPÜ.

**1.** Kollimator (1) zum Begrenzen eines Bündels energiereicher Strahlen (2), das von einer im wesentlichen punktförmigen Strahlungsquelle (3) ausgehend auf ein Behandlungsobjekt (4) gerichtet ist und insbesondere der stereotaktischen Konformationsbestrahlung von Tumoren dient, wobei der Kollimator (1) eine Scannvorrichtung mit einer Blende und einem Antrieb aufweist, welche ein Behandlungsobjekt (4) mittels der durch die Blende begrenzten Strahlen (2') abscannt,
**dadurch gekennzeichnet,**
**daß** der Kollimator (1) als strahlbegrenzendes Mittel eine Irisblende (5) mit mindestens drei Blendenblätter (6, 6', 6'' oder 7, 7', 7'', 7''' oder 8, 8', 8'', 8''', 8"" oder 9, 9', 9'', 9''', 9'''', 9''''') aufweist, die gleiche Winkel (α) einschließende, aneinanderliegende Seitenflächen (10) aufweisen, wobei die Blendenblätter (6, 6', 6" oder 7, 7', 7'', 7''' oder 8, 8', 8'', 8''', 8'''' oder 9, 9', 9'', 9''', 9'''', 9''''') eine strahlbegrenzende Öffnung (12) **dadurch** freigeben, daß eine Schiebebewegung (13) entlang der Seitenflächen (10) durch eine höchstens um eines verminderte Anzahl von Blendenblättern (6, 6', 6" oder 7, 7', 7", 7''' oder 8, 8', 8'', 8''', 8'''' oder 9, 9', 9'', 9''', 9'''', 9''''') stattfindet.

**2.** Kollimator nach Anspruch 1,
**dadurch gekennzeichnet,**
**daß** die Scannvorrichtung ein Roboterarm ist, wobei sich Strahlenquelle (3) und Irisblende (5) an dem Roboterarm befinden, der um das Behandlungsobjekt (4) bewegbar ist.

**3.** Kollimator nach Anspruch 1,
**dadurch gekennzeichnet,**
**daß** Strahlenquelle (3) und Kollimator (1) mittels einer Gantry in verschiedene Raumwinkelausrichtungen der durch den Kollimator (1) begrenzten Strahlen (2') zum Behandlungsobjekt (4) bringbar sind.

**4.** Kollimator nach einem der Ansprüche 1 bis 3,
**dadurch gekennzeichnet,**
**daß** seine Abschirmungsfähigkeit für energiereiche Strahlen einer Strahlungsquelle (3) im Megavoltbereich ausgelegt ist.

**5.** Kollimator nach Anspruch 4,
**dadurch gekennzeichnet,**
**daß** die Dicke der Blendenblätter (6, 6', 6'' oder 7, 7', 7'', 7''' oder 8, 8', 8'', 8''', 8'''' oder 9, 9', 9'', 9''', 9'''', 9''''') zwischen 6 und 10 cm liegt.

**6.** Kollimator nach einem der Ansprüche 1 bis 5,
**dadurch gekennzeichnet,**
**daß** eine Schiebebewegung (13) aller Blendenblätter (6, 6', 6'' oder 7, 7', 7'', 7''' oder 8, 8', 8'', 8''', 8"" oder 9, 9', 9'', 9''', 9'''', 9''''') um gleiche Stellwege (14) erfolgt, so daß nach der Stellbewegung die Öffnung (12) durch Teilbereiche (15) der Seitenflächen (10) gebildet wird, die gleiche Entfernungen vom Mittelpunkt (11) aufweisen.

**7.** Kollimator nach einem der Ansprüche 1 bis 6,
**dadurch gekennzeichnet,**
**daß** die Blendenblätter (6, 6', 6'' oder 7, 7', 7'', 7''' oder 8, 8', 8'', 8''', 8'''' oder 9, 9', 9'', 9''', 9'''', 9''''') mittels in Richtung der Schiebebewegung (13) verlaufender Linearführungen (16) gelagert sind.

**8.** Kollimator nach einem der Ansprüche 1 bis 7,
**dadurch gekennzeichnet,**
**daß** die Irisblende (5) vier Blendenblätter (7, 7', 7'', 7''') aufweist.

**9.** Kollimator nach Anspruch 8,
**dadurch gekennzeichnet,**
**daß** die jeweilige, die Öffnung (12) bildende Seitenfläche (10) an ihrem inneren Ende in einen nasenartig auskragenden einen Viertelkreis bildenden Kreisbogen (19) übergeht, so daß die vier Blendenblätter (7, 7', 7'', 7''') wahlweise eine runde Öffnung (17) oder quadratische Öffnungen mit gerundeten Ecken (18) verschiedenen Größen bilden können.

**10.** Kollimator nach einem der Ansprüche 1 bis 7,
**dadurch gekennzeichnet,**
**daß** die Irisblende (5) mindestens sechs Blendenblätter (9, 9', 9'', 9''', 9'''', 9''''') aufweist.

**11.** Kollimator nach einem der Ansprüche 1 bis 10,
**dadurch gekennzeichnet,**
**daß** Kraftbeaufschlagungen vorgesehen sind, die die Seitenflächen (10) der Blendenblätter (6, 6', 6'' oder 7, 7', 7'', 7''' oder 8, 8', 8'', 8''', 8''''' oder 9, 9', 9'', 9''', 9'''', 9''''') gegeneinanderdrücken.

**12.** Kollimator nach Anspruch 11,
**dadurch gekennzeichnet,**
**daß** auf die Blendenblätter (6, 6', 6" oder 7, 7', 7'', 7''' oder 8, 8', 8'', 8''', 8'''' oder 9, 9', 9'', 9''', 9'''', 9''''') Federn (20) wirken, die die Kraftbeaufschlagungen bewirken.

**13.** Kollimator nach Anspruch 11 oder 12,
**dadurch gekennzeichnet,**
**daß** die Seitenflächen (10) in ihren nicht zur Bildung einer Öffnung (12) dienenden aneinanderliegenden Bereichen gemeinsame Führungen (21) aufweisen, in denen die Seitenflächen (10) der aneinändergrenzenden Blendenblätter (6, 6', 6'' oder 7, 7', 7'', 7''' oder 8, 8', 8'', 8''', 8"" oder 9, 9', 9'', 9''', 9'''', 9''''') gegenseitig verschiebbar sind.

**14.** Kollimator nach einem der Ansprüche 1 bis 13,
**dadurch gekennzeichnet,**
**daß** die Schiebebewegung (13) der Blendenblätter (6, 6', 6'' oder 7, 7', 7'', 7''' oder 8, 8', 8'', 8''', 8'''' oder 9, 9', 9'', 9''', 9'''', 9''''') **dadurch** erfolgt, daß mindestens ein Blendenblatt (6, 6' oder 6''; 7, 7', 7" oder 7'''; 8, 8', 8'', 8''' oder 8''''; 9, 9', 9", 9''', 9"" oder 9''''') angetrieben wird.

**15.** Kollimator nach Anspruch 14,
**dadurch gekennzeichnet,**
**daß** alle Blendenblätter (6, 6', 6" oder 7, 7', 7'', 7''' oder 8, 8', 8'', 8''', 8'''' oder 9, 9', 9'', 9''', 9'''', 9''''') simultan angetrieben werden.

**16.** Kollimator nach Anspruch 15,
**dadurch gekennzeichnet,**
**daß** für jedes Blendenblatt (6, 6', 6" oder 7, 7', 7'', 7''' oder 8, 8', 8'', 8''', 8'''' oder 9, 9', 9'', 9''', 9'''', 9''''') ein Antrieb vorgesehen ist, wobei durch eine elektronische Steuerung eine simultane Bewegung erfolgt.

**17.** Kollimator nach Anspruch 15,
**dadurch gekennzeichnet,**
**daß** ein Antrieb über eine Mechanik (22) alle Blendenblätter (6, 6', 6'' oder 7, 7', 7'', 7''' oder 8, 8', 8'', 8''', 8'''' oder 9, 9', 9'', 9''', 9'''',9''''') simultan antreibt.

**18.** Kollimator nach Anspruch 17,
**dadurch gekennzeichnet,**
**daß** die Mechanik (22) die Blendenblätter (6, 6', 6'' oder 7, 7', 7'', 7''' oder 8, 8', 8'', 8''', 8'''' oder 9, 9', 9'', 9''', 9'''', 9''''') über, auf einer um den Mittelpunkt (11) drehbaren Kurvenscheibe (24) spiralförmig angeordnete Stellkurven (23) antreibt.

**19.** Kollimator nach Anspruch 17,
**dadurch gekennzeichnet,**
**daß** die Mechanik (22) ein um den Mittelpunkt (11) drehbares Stellorgan (25) aufweist, das auf jedes Blendenblatt (6, 6', 6'' oder 7, 7', 7'', 7''' oder 8, 8', 8'', 8''', 8'''' oder 9, 9', 9'', 9''', 9'''', 9''''') mit einem Stellarm (26) wirkt.

**20.** Kollimator nach Anspruch 19,
**dadurch gekennzeichnet,**
**daß** der Schiebebewegung (13) mittels der Stellarme (26) Rückstellfedern (27) entgegenwirken.

**21.** Kollimator nach einem der Ansprüche 1 bis 20,
**dadurch gekennzeichnet,**
**daß** die aneinanderliegenden Seitenflächen (10) der Blenden (6, 6', 6" oder 7, 7', 7'', 7''' oder 8, 8', 8'', 8''', 8'''' oder 9, 9', 9'', 9''', 9'''', 9''''') derart verlaufen, daß ein durch Toleranzen bedingter Spalt (28) nicht parallel zum Strahlengang verläuft.

**22.** Kollimator nach Anspruch 21,
**dadurch gekennzeichnet,**
**daß** die Seitenflächen (10) in Schieberichtung verlaufende komplementär ineinandergreifende Abweichungen bezüglich der Ebenheit der Seitenflächen (10) aufweisen.

**23.** Kollimator nach Anspruch 21,
**dadurch gekennzeichnet,**
**daß** die Irisblende (5) zu einer rechtwinklig zur optischen Achse liegenden gedachten Blendenebene (29) derart gekippt ist, daß ein Strahl (2) nicht mehr durch einen Spalt (28) fallen kann.

**24.** Kollimator nach einem der Ansprüche 1 bis 23,
**dadurch gekennzeichnet,**
**daß** sich außer der Irisblende (5) eine Festblende (30) für eine zusätzliche Abschirmung im Strahlengang befindet, deren Öffnung auf die größtmögliche Öffnung (12) der Irisblende (5) abgestimmt ist.
